(19) Europäisches Patentamt
European Patent Office
Office européen des brevets

(11) Publication number: 0 493 812 A1

(12) # EUROPEAN PATENT APPLICATION

(21) Application number: 91122307.1

(51) Int. Cl.[5]: C07D 241/08

(22) Date of filing: 27.12.91

(30) Priority: 27.12.90 JP 418592/90

(43) Date of publication of application: 08.07.92 Bulletin 92/28

(84) Designated Contracting States: BE DE FR GB NL

(71) Applicant: Ajinomoto Co., Ltd.
15-1, Kyobashi 1-chome, Chuo-ku
Tokyo(JP)

(72) Inventor: Toshihisa, Kato, c/o Central Res.Lab.
Ajinomoto Co., Inc., 1-1 Suzuki-cho, Kawasaki-ku
Kawasaki-shi, Kanagawa-ken(JP)
Inventor: Tadashi, Takemoto, c/o Central Res.Lab.
Ajinomoto Co., Inc., 1-1 Suzuki-cho, Kawasaki-ku
Kawasaki-shi, Kanagawa-ken(JP)

(74) Representative: Strehl, Schübel-Hopf, Groening
Maximilianstrasse 54 Postfach 22 14 55
W-8000 München 22(DE)

(54) **Process for preparing diketopiperazine derivatives.**

(57) A diketopiperazine derivative is prepared by reacting chloroacetylaspartic acid with ammonia water, diesterifying the resulting glycyl-aspartic acid with an alkanol, and then cyclizing the diester in neutral or weakly basic water or in an alkanol-water solvent mixture.

EP 0 493 812 A1

## BACKGROUND OF THE INVENTION

### Field of the Invention

The present invention relates to a process for preparing 2,5-dioxo-6-alkoxycarbonymethylpiperazines of the formula (I):

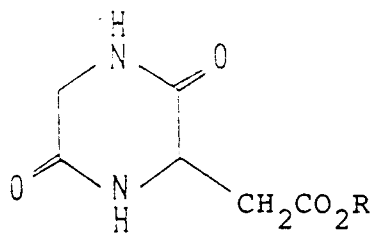

wherein R is alkyl, which are useful as intermediates for the synthesis of α-L-aspartyl-L-phenylalanine alkyl esters. α-L-Aspartyl-L-phenylalanine alkyl esters, especially the methyl esters, are well known because of their utility as sweeteners.

### Description of the Background

2,5-Dioxo-6-methoxycarbonylmethylpiperazine in the past has been prepared by a process of reacting dimethyl aspartate with a compound of formula (II):

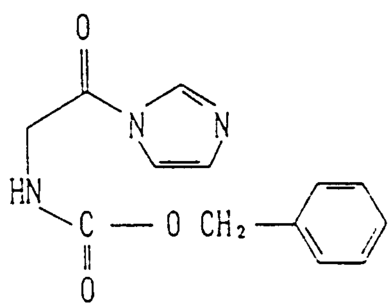

removing the protecting group from the intermediate with palladium on carbon, and then cyclizing the deprotected compound. [Research Disclosure (Res. Discl.), 281, 552].

However, this process has the drawback of requiring expensive reagents such as carbobenzoxychloride or carbonyldiimidazole which should be used for synthesis of compound (II), and accordingly the process is not necessarily appropriate for industrial production. A need therefore continues to exist for an industrially more satisfactory process for preparing diketopiperazine compounds.

## SUMMARY OF THE INVENTION

Accordingly, one object of the present invention is to provide a process for preparing 2,5-dioxo-6-alkoxycarbonylmethylpiperazines (I) from inexpensive aspartic acid under mild reaction conditions, using inexpensive reagents.

Briefly, the object and other object of the present invention as hereinafter will become more readily apparent can be attained in a method of preparing 2,5-dioxo-6-alkoxycarbonylmethylpiperazineby reacting chloroacetylaspartic acid with ammonia water, diesterifying the resulting glycylaspartic acid with an alkanol, and then cyclizing the diester in neutral or weakly basic water or in an alkanol-water solvent mixture.

## DETAILED DESCRIPTION OF THE PREFERRED EMBODIMENTS

The 2,5-dioxo-6-alkoxycarbonylmethylpiperazine compound of the invention has the formula:

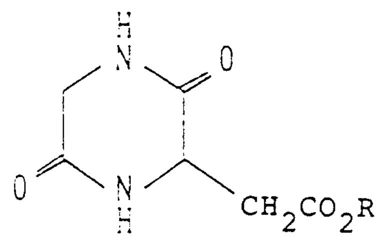

wherein R is a straight or branched lower alkyl of 1 to 6 carbon atoms such as methyl, ethyl, propyl, isopropyl, butyl, pentyl or hexyl, with particular preference for methyl.

As a result of investigation, it has now been found that by chloroacetylating aspartic acid, treating the resulting N-chloroacetylaspartic acid with ammonia water, then diesterifying the resulting glycyl-aspartic acid with hydrochloric acid and a lower alcohol and adjusting the pH to 6 to 8 to cyclize the diester, the desired diketopiperazine derivative of the invention can be obtained.

The aspartic acid starting material can be used in either its L- or D- form or as a racemic mixture.

For the chloroacetylation reaction of the present process, methods conventional to chloroacetylation, such as the Schotten-Baumann reaction using chloroacetyl chloride, may be used.

In general the N-chloroacetyl-aspartic acid intermediate obtained is crystallized only with difficulty, and is normally obtained as an oil. However, when it is allowed to stand over a long period of time (5 to 10 days), N-chloroacetyl-aspartic acid crystallizes and may be obtained as a solid. By reacting N-chloroacetyl-aspartic acid with ammonia water to form the amino compound, glycyl-aspartic

acid can be obtained. The reaction temperature is between 10°C and 40°C, preferably between 15°C and 25°C. The reaction time may vary depending upon the temperature, but the reaction is completed in about 6 hours at 15 to 25°C. In general, the reaction is carried out for 6 to 24 hours.

The ammonia may be used in a large molar excess, as long as the molar amount of ammonia is 4 times or more based on the amount of glycyl-aspartic acid. However, a molar amount of 5 to 50 times is preferred. The glycyl-aspartic acid which is prepared may be isolated over ion exchange resin or the like after excess ammonia is removed. Alternatively, glycyl-aspartic acid may be freeze-dried and may be used for the next step of esterification in the form of an ammonium salt. In this case, the esterification proceeds even if ammonium chloride is present as an impurity.

The esterification reaction is carried out in a solvent mixture of conc. hydrochloric acid and a lower alcohol or in dry hydrochloric acid and a lower alcohol, but the combination of dry hydrochloric acid and a lower alcohol is preferred. The reaction temperature is in the range of from 5°C to 60°C, preferably 15°C to 35°C.

The reaction can be followed by HPLC analysis. After the reaction is completed, the pH is adjusted to 6.0 to 8.0 and the reaction mixture is heated at 50 to 60°C for an hour to 3 hours without isolating glycyl-aspartic acid diester, whereby cyclization is easily performed to give product (I). The reaction solution is concentrated, if necessary, and cooled to crystallize the product. Thus, product (I) can be obtained in high purity.

From the diketopiperazine derivatives, α-L-aspartyl-L-phenylalanine methyl ester, which is useful as a sweetener, can be synthesized by the process described in Res. Discl. 281, 552.

Having generally described this invention, a further understanding can be obtained by reference to certain specific examples which are provided herein for purposes of illustration only and are not intended to be limiting unless otherwise specified.

## EXAMPLE 1

After 24 g (0.18 mol) of L-aspartic acid was suspended in 100 ml of water, 25 ml of 27% sodium hydroxide was added to the suspension to dissolve the acid. While stirring, 25 g (0.22 mol) of chloroacetyl chloride and 27% sodium hydroxide were added dropwise to the solution below 10°C in order to keep the pH in the range of 11.0 to 12.0. The amount of 27% sodium hydroxide required for completion of the dropwise addition was 77 ml. After completion of the addition of NaOH, the mixture was stirred for a further 30 minutes at 15°C. The pH was adjusted to 1.5 with 35% hydrochloric acid. In the reaction mixture was dissolved 20 g of sodium chloride, followed by extraction of the reaction mixture 3 times with 250 ml of ethyl acetate. The ethyl acetate extracts were combined, dried over anhydrous sodium sulfate and then concentrated to give 25 g of crude N-chloroacetyl-L-aspartic acid as an oil. HPLC analysis reveals that the purity was 94% (yield, 62%). The product was used for the next step of amination, as it was.

$^1$H NMR ($D_2O$) δ 3.00 (m, 2H), δ 4.08 (s, 2H), δ 4.84 (m, 1H)

## EXAMPLE 2

After 5 g (purity of 94%, 4.7 g, 0.022 mol, when calculated as N-chloroacetyl-L-aspartic acid) of crude N-chloroacetyl-L-aspartic acid obtained in Example 1 was dissolved in 30 ml of 28% ammonia water, the solution was allowed to stand at 20°C overnight. A 0.1 ml sample of the reaction solution was subjected to analysis. HPLC analysis shows that glycyl-L-aspartic acid was produced in a yield of 92% (based on N-chloroacetyl-L-aspartic acid).

After ammonia was removed by distillation under reduced pressure, the residue was freeze dried to give 5.5 g of crude glycyl-L-aspartic acid ammonium salt as a foamy solid. Analysis by HPLC reveals that the ammonium salt contained 3.80 g (0.02 mol) of glycyl-L-aspartic acid (yield 91%).

$^1$H NMR ($D_2O$) δ 2.50 (dd. 1H), δ 2.73 (dd. 1H), δ 2.73 (dd, 1H), δ 3.70 (s, 2H), δ 4.45 (m, 1H)

MS (FAB): 191 ($MH^+$)

## Example 3

After 5 g (purity of 94%, 4.7 g, when calculated as N-chloroacetyl-L-aspartic acid) of crude N-chloroacetyl-L-aspartic acid obtained in Example 1 was dissolved in 30 ml of 28% ammonia water, the solution was allowed to stand at 20°C overnight. After ammonia was removed by distillation under reduced pressure, desalting was performed using ion exchange resin. The desalted solution was concentrated to a volume of 10 ml and 50 ml of methanol was added thereto to crystallize glycyl-L-aspartic acid. The product was isolated by filtration. The amount of product obtained was 3.07 g and the yield was 72%.

$^1$H NMR ($D_2O$) δ 2.85 (m, 2H), δ 3.85 (s, 2H), δ 4.80 (m, 1H) MS (FAB): 191 ($MH^+$)

## EXAMPLE 4

After 5.5 g of crude glycyl-L-aspartic acid obtained in Example 2 was dissolved in 40 ml of methanol containing 0.5 g of dry hydrochloric acid,

the solution was reacted at 20 ° C for 6 hours. A 0.1 ml amount of reaction solution was subjected to analysis. Analysis by HPLC reveals that glycyl-L-aspartic acid dimethyl ester was produced in a yield of 87% (based on glycyl-L-aspartic acid).

EXAMPLE 5

After 20 ml of water was added to the reaction solution obtained in Example 4, the pH of the solution was adjusted to 7.0. Analysis by HPLC reveals that glycyl-L-aspartic acid dimethyl ester was produced in the amount of 3.79 g (0.017 mol).

After reacting the diester at 60 ° C for 3 hours, the solution was analyzed by HPLC. The desired 2,5-dioxo-6-methoxycarbonylmethylpiperazine was produced.

After the reaction solution was concentrated at 20 ml under reduced pressure and allowed to stand in a refrigerator overnight, precipitated crystals of 2,5-dioxo-6-methoxycarbonylmethylpiperazinewas formed which were isolated by filtration. The amount obtained was 2.28 g (0.012 mol) at a yield of 72% (based on glycyl-L-aspartic acid dimethyl ester).
$^1$H NMR ($d_6$-DMSO) δ 2.70 (m, 2H), δ 3.60 (s, 3H), δ 3.72 (m, 2H), δ 3.72 (m, 2H), δ 4.13 (m, 1H), δ 8.03 (brs, 1H), δ 8.10 (brs, 1H)
MS (FAB): 187 ($MH^+$), 373 ($2M+H^+$)

EXAMPLE 6

After 5.0 g (0.026 mol) of glycyl-L-aspartic acid was dissolved in 40 ml of methanol containing 0.5 g of dry hydrochloric acid, the solution was reacted at 25 ° C for 5 hours. After 20 ml of water was added to the reaction solution, the pH was adjusted to 7.0 with 27% sodium hydroxide. After reacting at 60 ° C for 3 hours, the mixture was concentrated to 20 ml under reduced pressure and allowed to stand in a refrigerator overnight. Precipitated crystals of 2,5-dioxo-6-methoxycarbonylmethylpiperazine were formed and were isolated by filtration. The amount obtained was 2.76 g at a yield of 57% (based on glycyl-L-aspartic acid).

EXAMPLE 7

After 5.0 g (0.026 mol) of glycyl-D-aspartic acid was dissolved in 40 ml of methanol containing 0.5 g of dry hydrochloric acid, the solution was reacted at 25 ° C for 5 hours. After 20 ml of water was added to the reaction solution, the pH was adjusted to 7.0 with 27% sodium hydroxide. After reacting at 60 ° C for 3 hours, the mixture was concentrated to 20 ml under reduced pressure and allowed to stand in a refrigerator overnight. Precipitated crystals of 2,5-dioxo-6-methoxycarbonylmethylpiperazine were formed and were isolated by filtration. The amount obtained was 2.62 g at a yield of 50% (based on glycyl-D-aspartic acid).
$^1$H NMR ($d_6$-DMSO) δ 1.22 (t, 3H), δ 2.72 (m, 2H), δ 3.75 (m, 2H), δ 4.10 (q, 2H), δ 4.13 (m, 1H)
MS (FAB): 201 ($MH^+$), 401 (2M + H + )

Having now fully described the invention, it will be apparent to one of ordinary skill in the art that many changes and modifications can be made thereto without departing from the spirit or scope of the invention as set forth herein.

**Claims**

**1.** A process for preparing a diketopiperazine derivative of the formula (I):

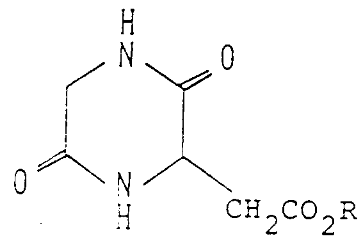

wherein R is a straight or branched lower alkyl of 1 to 6 carbon atoms, which comprises:

reacting chloroacetylaspartic acid with ammonia water; diesterifying the resulting glycylaspartic acid with an alkanol; and then

cyclizing the diester in neutral or weakly basic water or in an alkanol-water solvent mixture.

**2.** The process of Claim 1, wherein the reaction between chloroacetylaspartic acid and ammonia is conducted at 10 ° C to 40 ° C.

**3.** The process of Claim 2, wherein the reaction temperature ranges from 15 ° to 25 ° C.

**4.** The process of any of the Claims 1 to 3, wherein the reaction of chloroacetylaspartic acid and ammonia occurs for 6 to 24 hours.

**5.** The process of any of the Claims 1 to 4, wherein the molar amount of ammonia reacted with chloroacetylaspartic acid is 4 times or more based on the amount of glycyl-aspartic acid product.

**6.** The process of any of the Claims 1 to 5, wherein the glycyl-aspartic acid is esterified at a temperature of 5 ° C to 60 ° C.

**7.** The process of any of the Claims 1 to 6, wherein glycyl-aspartic acid is cyclized by heating at 50 to 60 ° C for about 1 to 3 hours.

European Patent Office

# EUROPEAN SEARCH REPORT

Application Number

EP 91 12 2307

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,P | WO-A-9 114 378 (THE NUTRASWEET COMPANY)<br>* page 4, line 30 - page 5, line 6 *<br>* page 8 - page 10, line 20; claims 56-58; examples 1-2B,3-5B *<br>--- | 1-7 | C07D241/08 |
| A | US-A-3 976 773 (A.C.W. CURRAN)<br>* column 2, line 48 - column 3, line 35 *<br>--- | 1-7 | |
| A | EP-A-0 216 746 (MONSANTO COMPANY)<br>* the whole document *<br>--- | 1-7 | |
| A | US-A-4 673 744 (K. HISAMITSU ET AL.)<br>* the whole document *<br>--- | 1-7 | |
| A,D | RESEARCH DISCLOSURE.<br>no. 281, 1987, HAVANT GB<br>page 552;<br>J.C. HUBBS: 'Synthesis of N-Acetyl-L-alpha-Aspartyl-L-Phenylalanine Methyl Ester'<br>* abstract *<br>--- | 1-7 | TECHNICAL FIELDS SEARCHED (Int. Cl.5) |
| A | AGRICULTURAL AND BIOLOGICAL CHEMISTRY.<br>vol. 52, no. 3, 1988, TOKYO JP<br>pages 819 - 827;<br>N. ISHIBASHI: 'A Mechanism for Bitter Taste Sensibility in Peptides'<br>* page 820; table 1 *<br>--- | 1-7 | C07D |
| A | JOURNAL OF THE AMERICAN CHEMICAL SOCIETY.<br>vol. 89, no. 4, 1967, GASTON, PA US<br>pages 988 - 998;<br>DELOS F. DETAR ET AL.: 'Sequence Peptide Polymers. I. Polymers based on Aspartic Acid and Glycine.'<br>*Abstract; CAS RN 13574-77-1*<br>----- | 1-7 | |

The present search report has been drawn up for all claims

| Place of search | Date of completion of the search | Examiner |
|---|---|---|
| THE HAGUE | 16 APRIL 1992 | P. BOSMA |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document